Europäisches Patentamt

European Patent Office

Office européen des brevets

(19)

(11) Veröffentlichungsnummer : **0 101 583**
**B1**

(12) # EUROPÄISCHE PATENTSCHRIFT

(45) Veröffentlichungstag der Patentschrift :
03.12.86

(21) Anmeldenummer : 83107659.1

(22) Anmeldetag : 03.08.83

(51) Int. Cl.⁴ : **A 61 B 10/00**

(54) **Ultraschall-Tomographiegerät.**

(30) Priorität : 19.08.82 DE 3230897

(43) Veröffentlichungstag der Anmeldung :
29.02.84 Patentblatt 84/09

(45) Bekanntmachung des Hinweises auf die Patenterteilung : 03.12.86 Patentblatt 86/49

(84) Benannte Vertragsstaaten :
DE FR GB NL

(56) Entgegenhaltungen :
EP-A- 0 018 771
EP-A- 0 032 751
WO-A-80 /001 93
WO-A-83 /000 09
GB-A- 2 088 556
US-A- 4 105 018
US-A- 4 222 274

(73) Patentinhaber : **Siemens Aktiengesellschaft Berlin
und München
Wittelsbacherplatz 2
D-8000 München 2 (DE)**

(72) Erfinder : **Hassler, Dieter
Flurweg 3
D-8521 Uttenreuth (DE)**
Erfinder : **Mittelstaedt, Wolfgang
Dina-Ernstberger-Strasse 28
D-8524 Neunkirchen (DE)**

EP 0 101 583 B1

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die Erfindung bezieht sich auf ein Ultraschall-Tomographiegerät mit einem Ultraschall-Sende- und Empfangssystem zur zeilenweisen Abtastung eines Untersuchungsobjektes aus verschiedenen Winkelrichtungen, einem Flüssigkeitstank für die Immersion des zu untersuchenden Objektes, einer Abdeckhaube für den Flüssigkeitstank mit einer zentralen runden Öffnung für die Führung des Untersuchungsobjektes und einem im Flüssigkeitstank um eine zur zentralen runden Öffnung in der Abdeckhaube ausgerichtete, im wesentlichen senkrechte Achse drehbaren Träger für ein Ultraschall-Sende- und -Empfangssystem.

Ultraschall-Tomographiegeräte lassen sich sowohl nach der Transmissions- als auch nach der Reflexionsmethode betreiben. Ein Ultraschall-Tomographiegerät für Transmissions-Tomographie (UCTT) ist z. B. aus der US-PS 41 05 018 vorbekannt. Ultraschall-Tomographiegeräte für Reflexions-Tomographie (UCTR) sind beispielsweise bekannt durch den Aufsatz « Resolution and Image Quality by Ultrasonic Echo Tomography : Experimental Approach » von E. Hundt, G. Maderlechner, E. Kronmüller und E. Trautenberg aus « Fifth International Symposium on Ultrasonic Imaging and Tissue Characterization and Second International Symposium on Ultrasonic Materials Characterization », June 1-6, 1980, Seite 7 oder auch durch den Aufsatz « Ultrasonic Reflectivity Tomography : Reconstruction with Circular Transducer Arrays » von Stephen J. Norton and Melvin Linzer aus « Ultrasonic Imaging 1 », 1979, Seiten 154 bis 184.

Mit bekannten Ultraschall-Tomographiegeräten, bei denen das Sendesystem an einem um eine senkrechte Achse drehbaren Träger um das Untersuchungsobjekt herumschwenkbar ist, lassen sich aus verschiedenen Richtungen, die durch Schwenkung des Trägers um die senkrechte Achse eingestellt werden können, horizontale Schnitte, sog. Coronar-Schnitte, durch ein in den Flüssigkeitstank eingetauchtes Untersuchungsobjekt, vorzugsweise der weiblichen Brust aber auch des männlichen Hodens, anfertigen. Um auf diese Weise das gesamte Untersuchungsobjekt erfassen zu können, muß nach jedem einzelnen Schnitt eine relative vertikale Verschiebung zwischen dem Sende- und dem Empfangssystem einerseits und dem Untersuchungsobjekt andererseits vorgenommen werden. Dabei sind Strukturveränderungen in vertikaler Richtung nur auf dem Umweg einer Informationsverarbeitung mindestens zweier benachbarter Schnitte ermittelbar.

Der Erfindung liegt die Aufgabe zugrunde, ein Ultraschall-Tomographiegerät zu entwickeln, das es erlaubt, zusätzliche, den räumlichen Verlauf von Strukturen aufzeigende Informationen in möglichst anschaulicher Form zu gewinnen.

Bei einem Ultraschall-Tomographiegerät der eingangs genannten Art ist daher erfindungsgemäß an einem zweiten, um die senkrechte Achse drehbaren, abgewinkelten Träger eine horizontale, die senkrechte Achse nur wenige Millimeter unter der Flüssigkeitsoberfläche schneidende weitere Achse befestigt, an der ein weiterer, im wesentlichen L-förmiger Träger schwenkbar aufgehängt ist, der an seinem freien Ende ein zweites Ultraschall-Sende- und -Empfangssystem trägt, das durch die Mittelsenkrechte auf die zentrale Öffnung in der Abdeckhaube hindurchschwenkbar und mit seiner Abstrahlrichtung auf den Mittelpunkt der zentralen Öffnung in der Abdeckhaube hin ausgerichtet ist. Mit einer solchen Anordnung von zwei in einem Flüssigkeitstank schwenkbaren Sende- und Empfangssystemen lassen sich intermittierend sowohl horizontale, sog. Coronar-Schnitte, in unterschiedlichen Ebenen als auch vertikale, um die Mittelsenkrechte auf das Untersuchungsobjekt gedrehte, sog. Sagittal-Schnitte, anfertigen. Die jeweils durch ein und denselben Punkt des Untersuchungsobjektes gehenden Coronar- und Sagittal-Schnitte vermitteln nicht nur ein dreidimensionales Bild der Strukturveränderung in ihren Schnittstellen. Sie ermöglichen zusätzlich die Kontrolle, ob im jeweils anderen System festgestellte Objekte real und nicht auf Artefakte zurückzuführen sind.

Um Untersuchungen der Mamma möglichst brustwandnah durchführen zu können, kann in besonders zweckmäßiger Weiterbildung der Erfindung an dem zweiten, um die senkrechte Achse drehbaren, abgewinkelten Träger ein, die horizontale Achse halbkreisförmig umschließender Zahnkranz befestigt sein und ein am L-förmigen Träger befestigter Motor ein mit dem Zahnkranz kämmendes Ritzel antreiben. Hierdurch wird es möglich, die horizontale Achse, um die das zweite Sende- und Empfangssystem schwenkbar ist, unmittelbar unter der Abdeckhaube der Auflagefläche für den Patienten, d. h. wenige Millimeter unterhalb der Flüssigkeitsoberfläche anzuordnen, weil alle für die Verstellung erforderlichen Bauelemente so ausnahmslos unterhalb der Ebene der horizontalen Achse untergebracht werden können. Auch erlaubt ein Zahnkranz, ander als ein Hebelgestänge, eine unabhängig von der jeweiligen Schwenklage gleichförmige Schwenkgeschwindigkeit des zweiten Sende- und Empfangssystems. Schließlich läßt sich dieser Antrieb äußerst platzsparend im Bereich des L-förmigen Trägers unterbringen.

In vorteilhafter Weiterbildung der Erfindung können die beiden, um eine senkrechte Achse drehbaren Träger in senkrechter Richtung verstellbar sein. Diese Verstellbarkeit ermöglicht es, Coronar-Schnitte unterschiedlicher Höhe ohne jede Höhenverstellung des Patienten vorzunehmen.

Eine zweckmäßige Konstruktion ergibt sich, wenn in weiterer Ausgestaltung der Erfindung das erste Ultraschall-Sende- und -Empfangssystem nach dem Prinzip der Ultraschall-Transmissions-Methode arbeitet und das zugehörige Detektorarray an dem zweiten, um die senkrechte Achse drehbaren,

abgewinkelten Träger befestigt ist. Bei dieser Konstruktion läßt sich die äußere Kontur des Detektorarrays des ersten Sende- und Empfangssystems exakt an den für die Schwenkung des zweiten Sende- und Empfangssystems um die horizontale Achse erforderlichen Freiraum anpassen.

Weitere Einzelheiten der Erfindung werden anhand eines in den Figuren dargestellten Ausführungsbeispieles näher erläutert. Es zeigen :

Figur 1 einen Schnitt durch ein erfindungsgemäßes Ultraschall-Tomographiegerät mit einer daraufliegenden Patientin,

Figur 2 eine Aufsicht auf den Flüssigkeitank mit dem darin angeordneten, umlaufenden Sende- und Empfangssystem bei abgenommener Abdeckhaube,

Figur 3 einen Schnitt längs der Linie III-III der Figur 1, und

Figur 4 eine schematische Darstellung der Schallkeulen zweier Ultraschallsender unterschiedlichen Durchmessers.

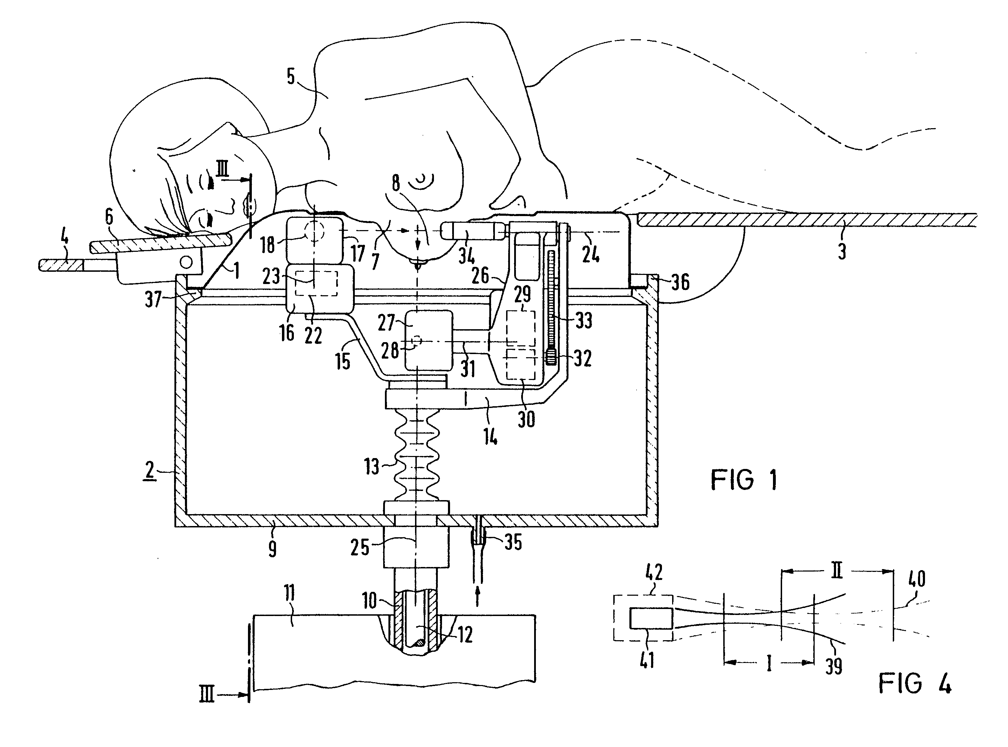

Die Figur 1 zeigt im Schnitt einen, durch eine Abdeckhaube 1 abgedeckten Flüssigkeitank 2 sowie eine Patientenlagerungsplatte 3, die im Bereich der Abdeckhaube ausgespart ist. Die Patientenlagerungsplatte trägt außerdem eine, über einen Handgriff 4, an die Lage der Patientin 5 anpaßbare Kopfstütze 6. Die Abdeckhaube besitzt eine runde zentrale Öffnung 7, durch die das Untersuchungsobjekt 8 zentriert in den Flüssigkeitank 2 eingeführt werden kann. Durch den Boden 9 des Flüssigkeitanks 2 hindurch ist eine Hohlwelle 10 geführt. Sie ist in nicht weiter dargestellter Weise mit einem unter dem Flüssigkeitank 2 angeordneten Antrieb 11 gekuppelt. In der Hohlwelle 10 ist eine weitere, in der Hohlwelle verdrehungssicher geführte Welle 12 axial verschiebbar. Diese innere Welle 12 ist über einen Gummibalg 13 abgedichtet. Sie ist mit zwei Trägern 14, 15 verschraubt, deren freie Schenkel nach gegenüberliegenden Seiten herausgeführt und nach oben abgewinkelt sind.

Auf dem in der Darstellung der Figur 1 linken abgewinkelten Träger 15 sind zwei wasserdichte Dosen 16, 17 übereinandergesetzt gehalten. Die obere, zylindrisch geformte Dose 17 trägt an ihrem Zylindermantel vier, um jeweils 90° gegeneinander versetzte Ultraschallwandler 18, 19, 20, 21 unterschiedlichen Durchmessers. In der unteren Dose 16 ist ein Motor 22 eingebaut, über den die obere Dose 17 um ihre Symmetrieachse 23 drehbar ist.

An dem anderen abgewinkelten Träger 14 ist unmittelbar unter der Abdeckhaube 1, d. h. wenige Millimeter unter der Oberfläche des Flüssigkeitsspiegels, eine horizontale Achse 24 befestigt, die so ausgerichtet ist, daß ihre Verlängerung die senkrechte Achse 25, um die die beiden Wellen 10 und 12 sowie die beiden abgewinkelten Träger 14, 15 drehbar sind, schneidet. An der horizontalen Achse 24 ist ein L-förmiger Träger 26 drehbar aufgehängt, der an seinem freien Ende eine zylindrische Dose 27 trägt. Auch diese zylindrische Dose 27 trägt an ihrem Umfang vier (nur einer sichtbar), um jeweils 90° gegeneinander versetzte Ultraschallwandler 28. Im L-förmigen Träger 26 sind zwei Motore 29, 30 eingebaut, von denen der eine Motor 29 die Dose 27 um ihre horizontale Symmetrieachse 31 dreht. Der andere Motor 30 treibt ein auf der gegenüberliegenden Seite des L-förmigen Trägers 26 vorstehendes Zahnritzel 32 an. Dieses Zahnritzel befindet sich mit einem am abgewinkelten Träger 14 befestigten, die horizontale Achse 24 halbkreisförmig umgebenden Zahnkranz 33 in Eingriff.

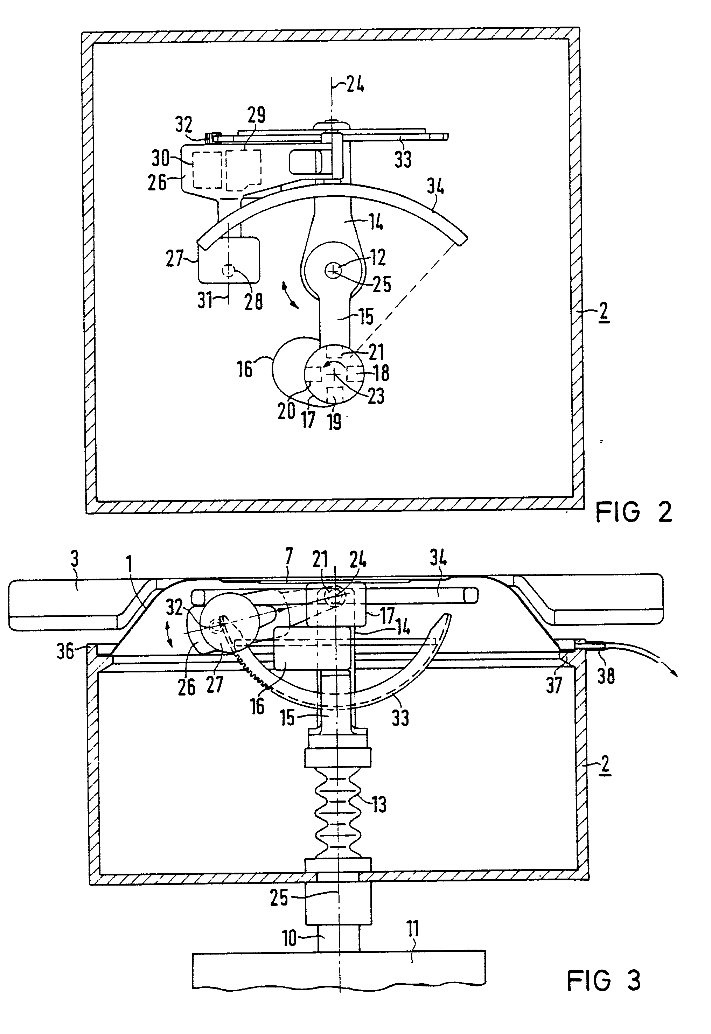

An der horizontalen Achse 24 ist außer dem L-förmigen Träger 26 auch ein halbkreisförmig gebogenes Detektorarray 34 befestigt. Dieses Detektorarray umgibt die am linken abgewinkelten Träger 15 gehalterte Dose 17 mit den vier Ultraschallwandlern nach Art eines um deren Symmetrieachse 23 geschlagenen Viertelkreises. Am Boden 9 des Flüssigkeitanks 2 ist ein Zulaufstutzen 35 für die Nachführung von Flüssigkeit zu erkennen. Wie die Figur 3 zeigt, überragt der Rand 36 des Flüssigkeitanks den Sitz 37 der Abdeckhaube 1 um einige Zentimeter. Im Rand ist ein Ablaufstutzen 38 für die aus der zentralen Öffnung 7 der Abdeckhaube austretende und in den Randbereich laufende Flüssigkeit.

Die Figur 2 zeigt eine Aufsicht auf die im Flüssigkeitank 2 angeordneten Bauelemente bei abgenommener Abdeckhaube 1. Man erkennt hier, daß die beiden abgewinkelten Träger 14, 15 einander gegenüberstehen. Die Figur 2 zeigt auch, daß das Detektorarray 34 wie ein Viertelkreis um die Symmetrieachse 23 der Dose 17 gewölbt ist. Ferner verdeutlicht die Figur 2 auch die Formgebung des L-förmigen Trägers 26. Dieser ist so ausgeformt, daß er bei der Schwenkung um die horizontale Achse 24 in seinen beiden Extremlagen — in den Darstellungen der Figuren 2 und 3 befindet er sich gerade in einer solchen — nicht mit dem Detektorarray 34 kollidieren kann.

In der Darstellung der Figur 3, die einen Schnitt längs der Linie III-III der Figur 1 darstellt, erkennt man die Form des halbkreisförmigen Zahnkranzes 33 und die Anordnung des Zahnritzels 32 am L-förmigen Träger 26. Auch verdeutlicht die Figur 3 die Lage, die das Detektorarray 34 einnehmen muß, wenn brustwandnahe Coronar-Schnitte anzufertigen sind. Auch die horizontale Achse 24, um die das zweite Sende- und Empfangssystem für die Erzeugung von Sagittal-Schnitten schwenkbar ist, ist hier angedeutet. Man sieht in dieser Figur besonders deutlich, daß kein Platz für über die Ebene der horizontalen Achse 23 aufragende Stellelemente zur Schwenkung des L-förmigen Trägers 26 verfügbar ist.

Soll mit dem Ultraschall-Tomographiegerät untersucht werden, so wird der Flüssigkeitank 2 durch Einpumpen von Flüssigkeit über den Zulaufstutzen 35 gefüllt. Sobald der Flüssigkeitank vollständig gefüllt ist, läuft die überschüssige Flüssigkeit an der zentralen Öffnung 7 der Abdeckhaube 1 über. Sie läuft an der Aussenwand der Abdeckhaube entlang in die Rinne, die sich zwischen dem überstehenden Rand 36 des Flüssigkeitanks 2 und dem Sitz 37 der Abdeckhaube auf dem Flüssigkeitank befindet.

Von dort läuft sie über den Ablaufstutzen 38 in ein Auffanggefäß (nicht dargestellt) ab.

Die zu untersuchende Patientin 5 kann sich so auf die Patientenlagerungsplatte 3 legen, daß die zu untersuchende Mamma 8 durch die zentrale Öffnung 7 in den Flüssigkeitstank 2 hineinhängt. Dabei kann sich die Patientin mit ihrem Kopf auf der Kopfstütze 6 abstützen. Bei der Inbetriebnahme des in der Figur 1 linken Ultraschall-Sendesystems wird beim Drehen der Dose 17 um ihre Symmetrieachse 23 nacheinander mit jedem der dort angebrachten vier Ultraschallwandler 18, 19, 20, 21 ein Schallbündel erzeugt, mit dem in horizontaler Richtung über das auf der gegenüberliegenden Seite des Untersuchungsobjektes an der horizontalen Achse 24 befestigte Detektorarray 34 gestrichen wird.

Weil die Beschallungskeulen 39, 40, wie die Figur 4 zeigt, einen, mit zunehmendem Durchmesser des Ultraschallwandlers 41, 42 weiter vom Ultraschallwandler entfernt liegenden, scharf gebündelten Bereich besitzen, lassen sich auch mit jedem der vier, in der Dose 17 untergebrachten Ultraschallwandler 18, 19, 20, 21 unterschiedlichen Durchmessers von unterschiedlich weit vom Ultraschallwandler entfernten Bereichen des Untersuchungsobjektes scharf lokalisierte Signale erhalten. Aus diesem Grunde wird die Dose 17 mit den vier Ultraschallwandlern nicht nur so weit geschwenkt, daß ein Ultraschallwandler mit seiner Schallkeule das Untersuchungsobjekt in seiner gesamten Breite überstreicht, sondern um etwa 360° um ihre Symmetrieachse 23 gedreht und so mit jedem Ultraschallwandler dieselbe Messung für jeweils einen anderen Entfernungsbereich wiederholt. Für jeden Entfernungsbereich werden die Signale jeweils eines anderen Ultraschallwandlers ausgewertet und wird mit den ausgewerteten Signalen ein horizontaler, sog. Coronar-Schnitt auf dem Leuchtschirm eines Sichtgerätes (nicht dargestellt) gebildet. Anschließend wird die in der Hohlwelle 10 längsverschiebliche Welle 12 abgesenkt, so daß die beiden abgewinkelten Träger 14, 15 um einige Millimeter tiefer gestellt sind. In dieser etwas tieferen Ebene wird der nächste Coronar-Schnitt in der oben geschilderten Weise wiederholt. So lassen sich mehrere parallele Coronar-Schnitte dicht untereinander durch das Untersuchungsobjekt legen.

Unabhängig von der Erstellung der Coronar-Schnitte kann der L-förmige Träger 26 mit der Dose 27 und den vier an deren Umfang angeordneten Ultraschallwandlern 28, die nach der Reflexions-Methode arbeiten, in der Darstellung der Figur 3 um nahezu 180° entgegen dem Uhrzeigersinn mit gleichbleibender Geschwindigkeit geschwenkt werden. Hierzu wird der das Zahnritzel 32 antreibende Motor 30 eingeschaltet. Dabei läuft das Zahnritzel längs des halbkreisförmigen Zahnkranzes 33 und nimmt dabei den L-förmigen Träger 26 mitsamt dem zweiten Ultraschall-Sende- und -Empfangssystem mit. Gleichzeitig mit dem das Zahnritzel antreibenden Motor 30 wird auch der andere, am L-förmigen Träger befestigte Motor 29 eingeschaltet. Dieser dreht die Dose 27 mit den vier an deren Umfang angebrachten Ultraschallwandlern um ihre horizontal ausgerichtete Symmetrieachse 31. Die Drehung der Dose erfolgt viel schneller als die Schwenkung des L-förmigen Trägers 26 längs des Zahnkranzes 22. So läßt sich das Untersuchungsobjekt aus verschiedenen Richtungen mit den vier Ultraschallwandlern der Dose 27 beschallen. Während der Schwenkbewegung des L-förmigen Trägers läßt sich so ein vertikaler Schnitt, ein sog. Sagittal-Schnitt, durch das Untersuchungsobjekt 8 legen. Diese Sagittal-Schnitte lassen sich durch Drehen der Hohlwelle 10 über deren Antrieb 11 in unterschiedlicher Richtung durch das Untersuchungsobjekt 8 legen. Durch Zusammenschau der jeweiligen Coronar-Schnitte mit den Sagittal-Schnitten lassen sich einerseits dreidimensionale Aussagen über den Verlauf bestimmter, im Schnittbereich der Coronar- und Sagittal-Schnitte liegender Objekte machen, lassen sich andererseits reale Bildpunkte von solchen, die durch Artefakte veranlaßt wurden und daher im jeweils anderen Schnittbild fehlen unterscheiden und lassen sich drittens über die Sagittal-Schnitte Aussagen über jene brustwandnahe Regionen treffen, die über die Coronar-Schnitte nicht erreichbar sind.

Bezugszeichenliste

1 Abdeckhaube
2 Flüssigkeitstank
3 Patientenlagerungsplatte
4 Handgriff
5 Patientin
6 Kopfstütze
7 Öffnung
8 Untersuchungsobjekt, Mamma
9 Boden
10 Hohlwelle
11 Antrieb
12 Welle
13 Gummibalg
14, 15 abgewinkelter Träger
16. 17 Dose
18. 19, 20, 21 Ultraschallwandler
22 Motor
23 Symmetrieachse
24 horizontale Achse

25 senkrechte Achse
26 L-förmiger Träger
27 Dose
28 Ultraschallwandler
29, 30 Motor
31 horizontale Symmetrieachse
32 Zahnritzel
33 Zahnkranz
34 Detektorarray
35 Zulaufstutzen
36 Rand
37 Sitz
38 Ablaufstutzen
39, 40 Beschallungskeule
41, 42 Ultraschallwandler

**Patentansprüche**

1. Ultraschall-Tomographiegerät mit einem Ultraschall-Sende- und -Empfangssystem zur zeilenweisen Abtastung eines Untersuchungsobjektes aus verschiedenen Winkelrichtungen, einem Flüssigkeitstank für die Immersion des zu untersuchenden Objektes, einer Abdeckhaube für den Flüssigkeitstank mit einer zentralen runden Öffnung für die Führung des Untersuchungsobjektes und einem im Flüssigkeitstank um eine zur zentralen runden Öffnung in der Abdeckhaube ausgerichtete, im wesentlichen senkrechte Achse drehbaren Träger für ein Ultraschall-Sende- und -Empfangssystem, dadurch gekennzeichnet, daß an einem zweiten, um die senkrechte Achse (25) drehbaren abgewinkelten Träger (14) eine horizontale, die senkrechte Achse nur wenige Millimeter unter der Flüssigkeitsoberfläche schneidende, weitere Achse (24) befestigt ist, an der ein weiterer, im wesentlichen L-förmiger Träger (26) schwenkbar aufgehängt ist, der an seinem freien Ende ein zweites Ultraschall-Sende- und -Empfangssystem trägt, das durch die Mittelsenkrechte auf die zentrale Öffnung (7) in der Abdeckhaube (1) hindurchschwenkbar und mit seiner Abstrahlrichtung auf den Mittelpunkt der zentralen Öffnung in der Abdeckhaube hin ausgerichtet ist.

2. Ultraschall-Tomographiegerät nach Anspruch 1, dadurch gekennzeichnet, daß an dem zweiten, um die senkrechte Achse (25) drehbaren, abgewinkelten Träger (14) ein, die horizontale Achse (24) halbkreisförmig umschließender Zahnkranz (33) befestigt ist und ein am L-förmigen Träger befestigter Motor (30) ein mit dem Zahnkranz kämmendes Zahnritzel (32) antreibt.

3. Ultraschall-Tomographiegerät nach Anspruch 1, dadurch gekennzeichnet, daß die beiden, um die senkrechte Achse (25) drehbaren Träger (14, 15) in senkrechter Richtung verstellbar sind.

4. Ultraschall-Tomographiegerät nach Anspruch 1, dadurch gekennzeichnet, daß das erste Ultraschall-Sende- und -Empfangssystem (17, 18, 19, 20, 21, 34) nach dem Prinzip der Ultraschall-Transmissions-Methode arbeitet und das zugehörige Detektorarray (34) an dem zweiten, um die senkrechte Achse (25) drehbaren abgewinkelten Träger (14) befestigt ist.

5. Ultraschall-Tomographiegerät nach Anspruch 4, dadurch gekennzeichnet, daß das Detektorarray (34) viertelkreisförmig um das erste Sendesystem (17 bis 21) gebogen und in der gleichen horizontalen Ebene angeordnet ist wie die Sendeachse.

6. Ultraschall-Tomographiegerät nach Anspruch 4, dadurch gekennzeichnet, daß das Detektorarray (34) an der horizontalen Achse (24) befestigt ist.

7. Ultraschall-Tomographiegerät nach Anspruch 1, dadurch gekennzeichnet, daß die beiden abgewinkelten Träger (14, 15) starr miteinander gekuppelt sind.

8. Ultraschall-Tomographiegerät nach Anspruch 3, dadurch gekennzeichnet, daß die Sende- und Empfangssysteme (17 bis 21, 34, 27, 28) von einer längs der senkrechten Achse (25) höhenverstellbaren und drehbaren Welle (12) getragen werden.

9. Ultraschall-Tomographiegerät nach Anspruch 1, dadurch gekennzeichnet, daß das zweite Ultraschall-Sende- und -Empfangssystem (27, 28) nach dem Prinzip der Ultraschall-Reflexions-Methode arbeitet.

**Claims**

1. An ultrasonic tomographic device with an ultrasonic transmitting and receiving system for scanning an object to be investigated line-by-line from different angular directions, a liquid tank in which the object to be investigated is immersed, the liquid tank having a covering cap with a central, round opening into which the object to be investigated is introduced, and with a carrier for an ultrasonic transmitting and receiving system, which carrier is rotatable about a fundamentally vertical axis aligned with the central, round opening in the covering cap, characterised in that a second angular carrier (14)

**0 101 583**

rotatable about the vertical axis (25) is attached to an axle (24) whose horizontal axis intersects the vertical axis only a few millimeters below the liquid surface, and from which an additional, fundamentally L-shaped carrier (26) is suspended to be pivotable and at its free end bears a second ultrasonic transmitting and receiving system which can be pivotted through the mean vertical to the central opening (7) in the covering cap (1) and whose radiation direction is aligned with the centre point of the central opening in the covering cap.

2. An ultrasonic tomographic device as claimed in Claim 1, characterised in that the second, angular carrier (14) rotatable about the vertical axis (25) is attached to a gear rim (33) which encloses the horizontal axle (24) in semi-circular fashion and a motor (30) attached to the L-shaped carrier drives a gear pinion (32) which engages with the gear rim.

3. An ultrasonic tomographic device as claimed in Claim 1, characterised in that the two carriers (14, 15) which are rotatable about the vertical axis (25) are adjustable in the vertical direction.

4. An ultrasonic tomographic device as claimed in Claim 1, characterised in that the first ultrasonic transmitting and receiving system (17, 18, 19, 20, 21, 34) operates in the ultrasonic transmission method, and the associated detector array (34) is attached to the second, angular carrier (14) which is rotatable about the vertical axis (25).

5. An ultrasonic tomographic device as claimed in Claim 4, characterised in that the detector array (34) is curved around the first transmitting system (17 to 21) in the shape of a quarter-circle and is arranged in the same horizontal plane as the transmitting axis.

6. An ultrasonic tomographic device as claimed in Claim 4, characterised in that the detector array (34) is attached to the horizontal axle (24).

7. An ultrasonic tomographic device as claimed in Claim 1, characterised in that the two angular carriers (14, 15) are rigidly coupled to one another.

8. An ultrasonic tomographic device as claimed in Claim 3, characterised in that the transmitting and receiving systems (17 to 21, 34, 27, 28) are rotatably supported by a shaft (12) which is height-adjustable along the vertical axis (25).

9. An ultrasonic tomographic device as claimed in Claim 1, characterised in that the second ultrasonic transmitting and receiving system (27, 28) operates in the ultrasonic reflection method.

**Revendications**

1. Appareil de tomographie par ultrasons, comprenant un système d'émission et de réception d'ultrasons pour l'exploration ligne par ligne sous différentes directions angulaires d'un objet à examiner, une cuve à liquide pour l'immersion de l'objet à examiner, un capot de recouvrement pour la cuve à liquide, pourvu d'une ouverture centrale ronde pour le guidage de l'objet à examiner, ainsi qu'un support pour un système d'émission et de réception d'ultrasons, disposé rotatif dans la cuve à liquide autour d'un axe essentiellement vertical aligné sur l'ouverture centrale ronde du capot, caractérisé en ce qu'un second support coudé (14), rotatif autour de l'axe vertical (25), porte un axe supplémentaire (24), horizontal, qui coupe l'axe vertical à quelques millimètres seulement sous la surface du liquide et auquel est suspendu oscillant un support supplémentaire (26), essentiellement en L, qui porte à son extrémité libre un second système d'émission et de réception d'ultrasons qui peut osciller à travers la verticale passant par le centre de l'ouverture centrale (7) du capot (1) et dont la direction de rayonnement est orientée sur le centre de l'ouverture centrale du capot.

2. Appareil selon la revendication 1, caractérisé en ce qu'une couronne dentée (33), entourant l'axe horizontal (24) en demi-cercle, est fixée au second support coudé (14), rotatif autour de l'axe vertical (25), et un moteur (30) fixé au support en L commande un pignon (32) engrenant avec la couronne dentée.

3. Appareil selon la revendication 1, caractérisé en ce que les deux supports (14, 15) rotatifs autour de l'axe vertical (25) sont déplaçables dans le sens vertical.

4. Appareil selon la revendication 1, caractérisé en ce que le premier système d'émission et de réception d'ultrasons (17, 18, 19, 20, 21, 34) opère selon le principe de la méthode de transmission des ultrasons et la rangée de détecteurs correspondant (34) est fixée au second support coudé (14), rotatif autour de l'axe vertical (25).

5. Appareil selon la revendication 4, caractérisé en ce que la rangée de détecteurs (34) est courbée en quart de cercle autour du premier système d'émission (17 à 21) et est disposée dans le même plan horizontal que l'axe d'émission.

6. Appareil selon la revendication 4, caractérisé en ce que la rangée de détecteurs (34) est fixée à l'axe horizontal (24).

7. Appareil selon la revendication 1, caractérisé en ce que les deux supports coudés (14, 15) sont rigidement couplés l'un à l'autre.

8. Appareil selon la revendication 3, caractérisé en ce que les systèmes d'émission et de réception (17 à 21, 34, 27, 28) sont portés par un arbre rotatif (12) qui est réglable en hauteur le long de l'axe vertical (25).

9. Appareil selon la revendication 1, caractérisé en ce que le second système d'émission et de réception d'ultrasons (27, 28) opère selon le principe de la méthode de réflexion d'ultrasons.

6

0 101 583

FIG 2

FIG 3

2